# EUROPEAN PATENT SPECIFICATION

(11) **EP 2 934 332 B1**
(45) Date of publication and mention of the grant of the patent: **24.10.2018**
(21) Application number: 13864696.3
(22) Date of filing: 20.12.2013
(51) Int. Cl.: A61B 8/12, A61B 8/14, A61B 8/08, A61B 8/00, A61B 5/00

(54) **ROTATIONAL IMAGING APPARATUS**
ROTIERENDE BILDGEBUNGSVORRICHTUNG
APPAREIL D'IMAGERIE ROTATIF

(30) Priority: 21.12.2012 US 201261740580 P
(43) Date of publication of application: 28.10.2015
(73) Proprietor: Fallon, Joseph, San Diego, CA 92130 (US); Fong, Lisa, San Diego, CA 92130 (US)
(72) Inventor: Fallon, Joseph, San Diego, CA 92130 (US); Fong, Lisa, San Diego, CA 92130 (US)
(74) Representative: de Haan, Poul Erik
(86) International application number: PCT/US2013/076791
(87) International publication number: WO 2014/100532

(56) References cited:
- US-A- 5 348 017
- US-A- 5 373 849
- US-A- 5 437 282
- US-A- 5 437 282
- US-A1- 2004 193 057
- US-A1- 2010 179 426
- US-B1- 6 344 037

## Description

### Field of the Invention

The invention generally relates to a rotational imaging apparatus and methods of use thereof.

### Background

Intravascular Ultrasound (IVUS) is an important interventional diagnostic procedure for imaging atherosclerosis and other vessel diseases and defects. In the procedure, an IVUS catheter is threaded over a guidewire into a blood vessel, and images are acquired of the atherosclerotic plaque and surrounding area using ultrasonic echoes. That information is much more descriptive than information from other imaging techniques, such as angiography, which shows only a two-dimensional shadow of a vessel lumen.

There are two types of IVUS catheters commonly in use, mechanical/rotational IVUS catheters and solid state catheters. A solid state catheter (or phased array) has no rotating parts, but instead includes an array of transducer elements (for example 64 elements). In a rotational IVUS catheter, a single transducer having a piezoelectric crystal is rapidly rotated (e.g., at approximately 1800 revolutions per minute) while the transducer is intermittently excited with an electrical pulse. The excitation pulse causes the transducer to vibrate, sending out a series of transmit pulses. The transmit pulses are sent at a frequency that allows time for receipt of echo signals. The sequence of transmit pulses interspersed with receipt signals provides the ultrasound data required to reconstruct a complete cross-sectional image of a vessel.

Typically, rotational IVUS catheters have a drive cable disposed within a catheter body. A transducer is attached to a distal end of the drive cable. A coaxial cable is disposed within the drive cable and also couples to the transducer. The coaxial cable delivers the intermittent electrical transmit pulses to the transducer, and delivers the received electrical echo signals from the transducer to the receiver amplifier. The IVUS catheter is removably coupled to an interface module, which controls the rotation of the drive cable and the coaxial cable within the catheter body and contains the transmitter and receiver circuitry for the transducer.

Maintaining the coaxial cable in a relaxed state is important for producing a high quality image. A problem with rotational IVUS catheters is that the coaxial cable becomes stressed during operation due to the coaxial cable rotating at a different rate than the drive cable, which results in kinking or winding of the coaxial cable. Stress on the coaxial cable results in poor data acquisition or loss of data, both of which lead to poor image quality.

A drive shaft for acoustic imaging catheters and flexible catheters is disclosed in document US 5 437 282 A.

### Summary

The invention generally provides rotational imaging apparatuses that are configured to prevent kinking or winding of a signal transmission member in the apparatus. Apparatuses of the invention include a hollow elongate body. The hollow body includes a proximal end and a distal end, and the proximal end includes a connector so that the apparatus can be connected to an interface module. A rotatable drive member is disposed within the body, and a rotatable electrical signal transmission member is disposed within the drive member. The drive member and the electrical signal transmission member are coupled to an imaging device within the body. Aspects of the invention are accomplished by additionally attaching a portion of the electrical signal transmission member to the drive member distal to the connector. Such attachment stabilizes the electrical signal transmission member and helps synchronize rotation of the electrical signal transmission member with the drive member, thereby preventing the electrical signal transmission member from becoming stressed due to kinking or winding. Maintaining the electrical signal transmission member in a relaxed state prevents loss of data or acquisition of poor data, thus leading to production of high quality images.

Typically, the imaging device is coupled to a distal end of the drive member. The signal transmission member can be attached to the drive member at any point that is distal to the connector and before the imaging device. In certain embodiments, the signal transmission member is attached to the drive member along a proximal portion of the drive member, e.g., immediately after the connector. Attachment may be by any method known in the art, and preferred methods use an adhesive, such as an epoxy. The signal transmission member may be any elongate structure that is capable of transmitting electrical signals. In certain embodiments, the signal transmission member is coaxial cable.

Any imaging device known in the art may be used with apparatuses of the invention. Exemplary devices include ultrasound devices and optical coherence tomography (OCT) devices. In certain embodiments, the imaging device is an ultrasound device and the imaging device includes an ultrasound transducer. Typically, ultrasound systems rely on conventional piezoelectric transducers, built from piezoelectric ceramic (commonly referred to as the crystal) and covered by one or more matching layers (typically thin layers of epoxy composites or polymers). Two advanced transducer technologies that have shown promise for replacing conventional piezoelectric devices are the PMUT (Piezoelectric Micromachined Ultrasonic Transducer) and CMUT (Capacitive Micromachined Ultrasonic Transducer). PMUT and CMUT transducers may provide improved image quality over that provided by the conventional piezoelectric transducer.

Generally, the apparatus may connect to an interface module via the connector. The interface module typically includes components necessary for rotating the drive member and the electrical signal transmission member. Apparatuses of the invention may additionally include an elongate catheter. In those embodiments, the body is configured to fit within the catheter. Apparatuses of the invention are configured from insertion in a vessel lumen, and include additional features that facilitate operation within the vessel. For example, a distal end of the body may include an atraumatic tip. The atraumatic tip is configured to guide the apparatus through the vessel lumen while avoiding perforation of the lumen. Additionally, the body, the drive member and the signal transmission member may be flexible so that the apparatus may more easily be advanced through the vessel.

Other aspects of the invention provide methods for imaging a vessel lumen. Such methods involve providing a rotational imaging apparatus that includes a hollow elongate body. The hollow body includes a proximal and a distal end, and the proximal end includes a connector. A rotatable drive member is disposed within the body, and a rotatable electrical signal transmission member is disposed within the drive cable. The drive member and the electrical signal transmission member are coupled to an imaging device within the body and a portion of the electrical signal transmission member is attached to the drive member distal to the connector. The apparatus is inserted into a vessel lumen and used to obtain image data of the vessel lumen.

### Brief Description of the Drawings

FIG. 1 is a simplified fragmentary diagrammatic view of a rotational IVUS probe.
FIG. 2 is a simplified fragmentary diagrammatic view of an interface module and catheter for the rotational IVUS probe of FIG. 1 incorporating basic ultrasound transducer technology.
FIG. 3 is a simplified fragmentary diagrammatic view of a rotational IVUS probe in which a portion of a signal transmission member is attached to a drive member.
FIGS. 4-6 are fragmentary diagrammatic views of the rotational IVUS probe illustrating different embodiments in which a portion of a signal transmission member is attached to a drive member.

### Detailed Description

The invention generally relates to rotational imaging apparatuses and methods of use thereof. In certain aspects, the apparatus includes a hollow elongate body. The hollow body includes a proximal and a distal end, and the proximal end includes a connector. A rotatable drive member is disposed within the body, and a rotatable electrical signal transmission member is disposed within the drive cable. The drive member and the electrical signal transmission member are coupled to an imaging device within the body and a portion of the electrical signal transmission member is attached to the drive member distal to the connector.

Typically, apparatuses of the invention are provided in the form of a catheter. Any imaging device known in the art may be used with apparatuses of the invention. Exemplary devices include ultrasound devices and optical coherence tomography (OCT) devices. In particular embodiments, the imaging device is an ultrasound device and apparatuses of the invention are intravascular ultrasound (IVUS) catheters. The general design and construction of such catheters is shown, for example in Yock, U.S. Pat. Nos. 4,794,931, 5,000,185, and 5,313,949; Sieben et al., U.S. Pat. Nos. 5,243,988, and 5,353,798; Crowley et al., U.S. Pat. No. 4,951,677; Pomeranz, U.S. Pat. No. 5,095,911, Griffith et al., U.S. Pat. No. 4,841,977, Maroney et al., U.S. Pat. No. 5,373,849, Born et al., U.S. Pat. No. 5,176,141, Lancee et al., U.S. Pat. No. 5,240,003, Lancee et al., U.S. Pat. No. 5,375,602, Gardineer et at., U.S. Pat. No. 5,373,845, Seward et al., Mayo Clinic Proceedings 71(7):629-635 (1996), Packer et al., Cardiostim Conference 833 (1994), "Ultrasound Cardioscopy," Eur. J.C.P.E. 4(2):193 (June 1994), Eberle et al., U.S. Pat. No. 5,453,575, Eberle et al., U.S. Pat. No. 5,368,037, Eberle et at., U.S. Pat. No. 5,183,048, Eberle et al., U.S. Pat. No. 5,167,233, Eberle et at., U.S. Pat. No. 4,917,097, Eberle et at., U.S. Pat. No. 5,135,486, and other references well known in the art relating to intraluminal ultrasound devices and modalities. The catheter will typically have proximal and distal regions, and will include an imaging tip located in the distal region. Such catheters have an ability to obtain echographic images of the area surrounding the imaging tip when located in a region of interest inside the body of a patient. The catheter, and its associated electronic circuitry, will also be capable of defining the position of the catheter axis with respect to each echographic data set obtained in the region of interest.

Besides intravascular ultrasound, other types of ultrasound catheters can be made using the teachings provided herein. By way of example and not limitation, other suitable types of catheters include non-intravascular intraluminal ultrasound catheters, intracardiac echo catheters, laparoscopic, and interstitial catheters. In addition, the probe may be used in any suitable anatomy, including, but not limited to, coronary, carotid, neuro, peripheral, or venous.

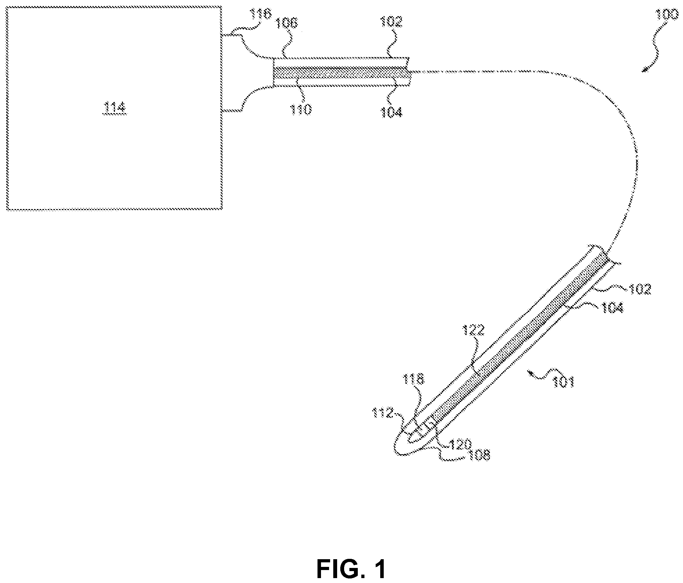

FIG. 1 shows a rotational intravascular ultrasound probe 100 for insertion into a patient for diagnostic imaging. The probe 100 includes a catheter 101 having a catheter body 102 and a hollow transducer shaft 104. The catheter body 102 is flexible and has both a proximal end portion 106 and a distal end portion 108. The catheter body 102 may be a single lumen polymer extrusion, for example, made of polyethylene (PE), although other polymers may be used. Further, the catheter body 102 may be formed of multiple grades of PE, for example, HDPE and LDPE, such that the proximal portion exhibits a higher degree of stiffness relative to the mid and distal portions of the catheter body. This configuration provides an operator with catheter handling properties required to efficiently perform the desired procedures.

The catheter body 102 is a sheath surrounding the transducer shaft 104. For explanatory purposes, the catheter body 102 in FIG. 1 is illustrated as visually transparent such that the transducer shaft 104 disposed therein can be seen, although it will be appreciated that the catheter body 102 may or may not be visually transparent. The transducer shaft 104 is flushed with a sterile fluid, such as saline, within the catheter body 102. A fluid injection port (not shown) may be supplied at a junction of the catheter body 102 to the interface module so that the space inside the catheter body 102 can be flushed initially and periodically. The fluid serves to eliminate the presence of air pockets around the transducer shaft 104 that adversely affect image quality. The fluid can also act as a lubricant. The transducer shaft 104 has a proximal end portion 110 disposed within the proximal end portion 106 of the catheter body 102 and a distal end portion 112 disposed within the distal end portion 108 of the catheter body 102.

The distal end portion 108 of the catheter body 102 and the distal end portion 112 of the transducer shaft 104 are inserted into a patient during the operation of the probe 100. The usable length of the probe 100 (the portion that can be inserted into a patient) can be any suitable length and can be varied depending upon the application. The distal end portion 112 of the transducer shaft 104 includes a transducer subassembly 118.

The transducer subassembly 118 is used to obtain ultrasound information from within a vessel. It will be appreciated that any suitable frequency and any suitable quantity of frequencies may be used. Exemplary frequencies range from about 5 MHz to about 80 MHz. Generally, lower frequency information (e.g., less than 40 MHz) facilitates a tissue versus blood classification scheme due to the strong frequency dependence of the backscatter coefficient of the blood. Higher frequency information (e.g., greater than 40 MHz) generally provides better resolution at the expense of poor differentiation between blood speckle and tissue, which can make it difficult to identify the lumen border. Blood speckle reduction algorithms such as motion algorithms (such as ChromaFlo, Q-Flow, etc.), temporal algorithms, harmonic signal processing, can be used to enhance images where light back scattered from blood is a problem.

The proximal end portion 106 of the catheter body 102 and the proximal end portion 110 of the transducer shaft 104 are connected to an interface module 114 (sometimes referred to as a patient interface module or PIM). The proximal end portions 106, 110 are fitted with a catheter hub 116 that is removably connected to the interface module 114.

The catheter body 102 may include a flexible atraumatic distal tip. For example, an integrated distal tip can increase the safety of the catheter by eliminating the joint between the distal tip and the catheter body. The integral tip can provide a smoother inner diameter for ease of tissue movement into a collection chamber in the tip. During manufacturing, the transition from the housing to the flexible distal tip can be finished with a polymer laminate over the material housing. No weld, crimp, or screw joint is usually required. The atraumatic distal tip permits advancing the catheter distally through the blood vessel or other body lumen while reducing any damage caused to the body lumen by the catheter. Typically, the distal tip will have a guidewire channel to permit the catheter to be guided to the target lesion over a guidewire. In some exemplary configurations, the atraumatic distal tip includes a coil. In some configurations the distal tip has a rounded, blunt distal end. The catheter body can be tubular and have a forward-facing circular aperture which communicates with the atraumatic tip.

The rotation of the transducer shaft 104 within the catheter body 102 is controlled by the interface module 114, which provides a plurality of user interface controls that can be manipulated by a user. The interface module 114 also communicates with the transducer subassembly 118 by sending and receiving electrical signals to and from the transducer subassembly 118 via at least one electrical signal transmission member (e.g., wires or coaxial cable) within the transducer shaft 104. The interface module 114 can receive, analyze, and/or display information received through the transducer shaft 104. It will be appreciated that any suitable functionality, controls, information processing and analysis, and display can be incorporated into the interface module 114. Further description of the interface module is provided, for example in Corl (U.S. patent application number 2010/0234736).

The transducer shaft 104 includes a transducer subassembly 118, a transducer housing 120, and a drive member 122. The transducer subassembly 118 is coupled to the transducer housing 120. The transducer housing 120 is attached to the drive member 122 at the distal end portion 112 of the transducer shaft 104. The drive member 122 is rotated within the catheter body 102 via the interface module 114 to rotate the transducer housing 120 and the transducer subassembly 118. The transducer subassembly 118 can be of any suitable type, including but not limited to one or more advanced transducer technologies such as PMUT or CMUT. The transducer subassembly 118 can include either a single transducer or an array.

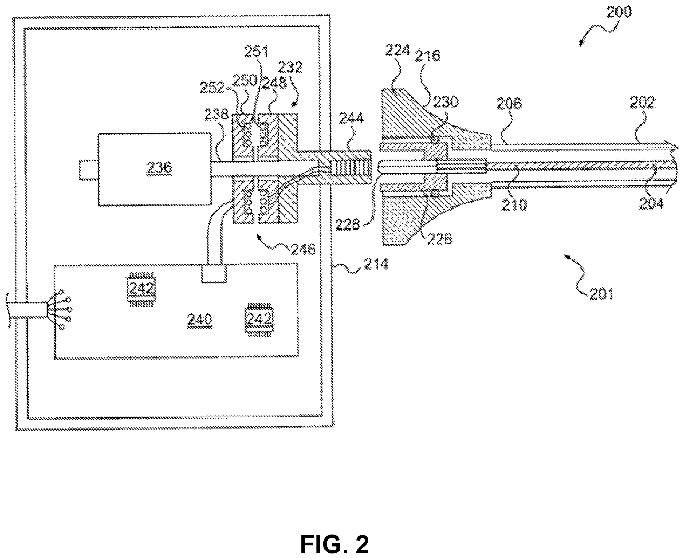

FIG. 2 shows a rotational IVUS probe 200 utilizing a common spinning element 232. The probe 200 has a catheter 201 with a catheter body 202 and a transducer shaft 204. As shown, the catheter hub 216 is near the proximal end portion 206 of the catheter body 202 and the proximal end portion 210 of the transducer shaft 204. The catheter hub 216 includes a stationary hub housing 224, a dog 226, a connector 228, and bearings 230. The dog 226 mates with a spinning element 232 for alignment of the hub 216 with the interface module 214 and torque transmission to the transducer shaft 204. The dog 226 rotates within the hub housing 224 utilizing the bearings 230. The connector 228 in this figure is coaxial. The connector 228 rotates with the spinning element 232, described further herein.

As shown, the interior of the interface module 214 includes a motor 236, a motor shaft 238, a printed circuit board (PCB) 240, the spinning element 232, and any other suitable components for the operation of the IVUS probe 200. The motor 236 is connected to the motor shaft 238 to rotate the spinning element 232. The printed circuit board 240 can have any suitable number and type of electronic components 242, including but not limited to the transmitter and the receiver for the transducer.

The spinning element 232 has a complimentary connector 244 for mating with the connector 228 on the catheter hub 216. As shown, the spinning element 232 is coupled to a rotary portion 248 of a rotary transformer 246. The rotary portion 248 of the transformer 246 passes the signals to and from a stationary portion 250 of the transformer 246. The stationary portion 250 of the transformer 246 is wired to the transmitter and receiver circuitry on the printed circuit board 240.

The transformer includes an insulating wire that is layered into an annular groove to form a two- or three-turn winding. Each of the rotary portion 250 and the stationary portion 248 has a set of windings, such as 251 and 252 respectively. Transformer performance can be improved through both minimizing the gap between the stationary portion 250 and the rotary portion 248 of the transformer 246 and also by placing the windings 251, 252 as close as possible to each other.

A problem that occurs during operation of rotational IVUS catheters is that the signal transmission member tends to become kinked or wound around the drive member. That causes stress on the signal transmission member, which leads to acquisition of poor image data or loss of data. That problem is solved by providing an additional attachment of the signal transmission member to the drive member. Such attachment stabilizes the electrical signal transmission member and helps synchronize rotation of the electrical signal transmission member with the drive member, thereby preventing the electrical signal transmission member from becoming stressed due to kinking or winding.

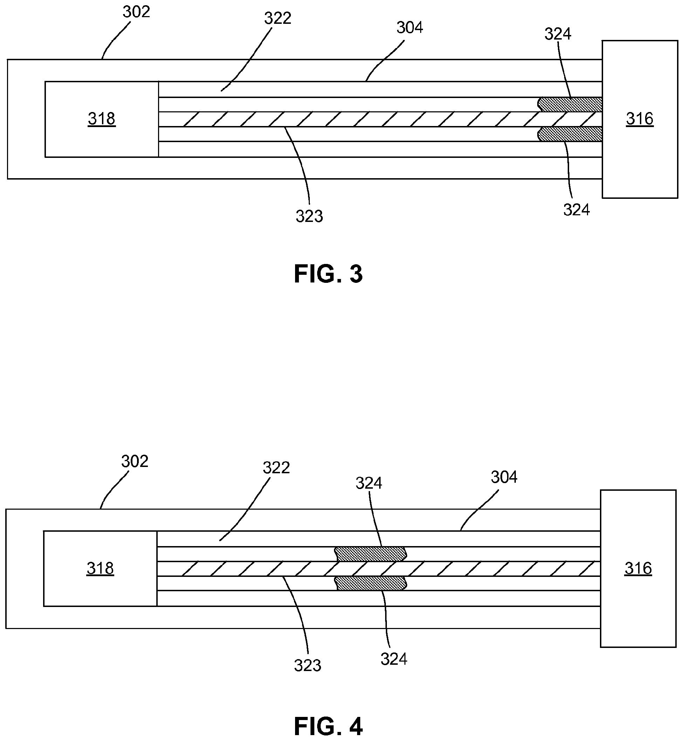

FIG. 3 shows a rotational IVUS probe 300 having a portion of the signal transmission member 323 attached to the drive member 322. In that figures, there is a catheter body 302 and an elongate transducer shaft 304 within catheter body 302. A distal end of the transducer shaft 304 is coupled to a transducer subassembly 318. A proximal end of the transducer shaft 304 is coupled to a connector hub 316. A drive member 322 is disposed within the transducer shaft 304 and is coaxial with the transducer shaft 304. A distal end of the drive member 322 is coupled to the transducer subassembly 318, and a proximal end of the drive member 322 is coupled to the connector hub 316. A signal transmission member 323 is disposed within the drive member 322 and is coaxial with the drive member 322. A distal end of the signal transmission member 323 is coupled to the transducer subassembly 318, and a proximal end of the signal transmission member 323 is coupled to the connector hub 316. The signal transmission member 323 delivers the intermittent electrical transmit pulses to the transducer subassembly 318 from the interface module, and delivers the received electrical echo signals from the transducer subassembly 318 to the receiver amplifier in the interface module.

To prevent winding or kinking of the signal transmission member 323, a portion of the signal transmission member 323 is attached to the drive member 322 distal to the connector hub 316. That is shown in FIG. 3 as darkened sections 324 on each side of signal transmission member 323. An exemplary attachment is shown in FIG. 3, where the portion of the signal transmission member 323 is attached to the drive member 323 immediately after the connector hub 316. However, the embodiment shown is FIG. 3 is only illustrative and the invention is not limited to that embodiment.

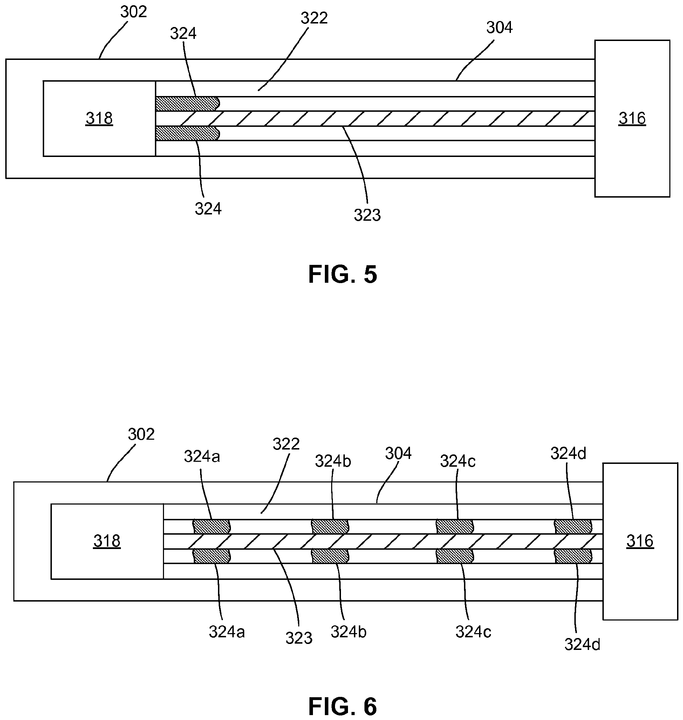

The attachment may be at any point between the transducer subassembly 318 and the connector hub 316. For example, as shown in FIG. 4, the signal transmission member 323 is attached to the drive member 322 at a position that is approximately halfway between the connector hub 316 and the transducer subassembly 318. As shown in FIG. 5, the signal transmission member 323 is attached to the drive member 322 at a position immediately after the transducer subassembly 318.

In certain embodiments, more than one attachment point is used, such as two attachment points, three attachment points, four attachment points, five attachment points, 10 attachment points, 20 attachment points, etc. The number attachment points will depend on the length on the apparatus and the anatomy of the vessel. For example, as shown in FIG. 6, multiple attachment points (324a, 324b, 324c, and 324d) are used to attach the transmission member 323 to the drive member 322. Similarly, the length of each of the attachments in FIGS. 3-6 is merely illustrative, and is not meant to be limiting. It should be understood that the attachment points 324 can be longer or shorter depending on the length on the apparatus and the anatomy of the vessel.

Additionally, FIG. 3 illustrates only a single attachment approach, in which the signal transmission member 323 is attached and coaxially centered with respect to the drive member 322. Other attachment positions are within the scope of the invention, and the attachment is not required to be such that the signal transmission member 323 is centered with respect to the drive member 322. For example, the attachment could be biased or the signal transmission member 323 could be attached to an inner wall of the drive member 322.

Attachment may be by any methods known in the art. In certain embodiments, an adhesive such as an epoxy is used to attach the signal transmission member 323 to the drive member 322. Additional exemplary adhesives are commercially available under the trade name LOCTITE. Other adhesives include thermo-setting plastics, UV curable adhesives, or silicone rubber gels. Generally, prior to attaching the connector hub 116, the adhesive is injected into the space(s) between the signal transmission member 323 and the drive member 322. The adhesive is allowed to cure, securing a portion of the signal transmission member 323 to the drive member 322. Then, the connector hub 116 is attached to the apparatus.

As previously described herein, other imaging devices, in addition, or alternatively, to IVUS devices, may be used with apparatuses of the invention. One exemplary device includes optical coherence tomography (OCT) devices. OCT is a medical imaging methodology using a miniaturized near infrared light-emitting probe. As an optical signal acquisition and processing method, it captures micrometer-resolution, three-dimensional images from within optical scattering media (e.g., biological tissue). Recently it has also begun to be used in interventional cardiology to help diagnose coronary artery disease. OCT allows the application of interferometric technology to see from inside, for example, blood vessels, visualizing the endothelium (inner wall) of blood vessels in living individuals.

OCT systems and methods are generally described in Castella et al., U.S. Patent No. 8,108,030, Milner et al., U.S. Patent Application Publication No. 2011/0152771, Condit et al., U.S. Patent Application Publication No. 2010/0220334, Castella et al., U.S. Patent Application Publication No. 2009/0043191, Milner et al., U.S. Patent Application Publication No. 2008/0291463, and Kemp, N., U.S. Patent Application Publication No. 2008/0180683.

In OCT, a light source delivers a beam of light to an imaging device to image target tissue. Light sources can include pulsating light sources or lasers, continuous wave light sources or lasers, tunable lasers, broadband light source, or multiple tunable laser. Within the light source is an optical amplifier and a tunable filter that allows a user to select a wavelength of light to be amplified. Wavelengths commonly used in medical applications include near-infrared light, for example between about 800 nm and about 1700 nm.

Aspects of the invention may obtain imaging data from an OCT system, including OCT systems that operate in either the time domain or frequency (high definition) domain. Basic differences between time-domain OCT and frequency-domain OCT is that in time-domain OCT, the scanning mechanism is a movable mirror, which is scanned as a function of time during the image acquisition. However, in the frequency-domain OCT, there are no moving parts and the image is scanned as a function of frequency or wavelength.

In time-domain OCT systems an interference spectrum is obtained by moving the scanning mechanism, such as a reference mirror, longitudinally to change the reference path and match multiple optical paths due to reflections within the sample. The signal giving the reflectivity is sampled over time, and light traveling at a specific distance creates interference in the detector. Moving the scanning mechanism laterally (or rotationally) across the sample produces two-dimensional and three-dimensional images.

In frequency domain OCT, a light source capable of emitting a range of optical frequencies excites an interferometer, the interferometer combines the light returned from a sample with a reference beam of light from the same source, and the intensity of the combined light is recorded as a function of optical frequency to form an interference spectrum. A Fourier transform of the interference spectrum provides the reflectance distribution along the depth within the sample.

Several methods of frequency domain OCT are described in the literature. In spectral-domain OCT (SD-OCT), also sometimes called "Spectral Radar" (Optics letters, Vol. 21, No. 14 (1996) 1087-1089), a grating or prism or other means is used to disperse the output of the interferometer into its optical frequency components. The intensities of these separated components are measured using an array of optical detectors, each detector receiving an optical frequency or a fractional range of optical frequencies. The set of measurements from these optical detectors forms an interference spectrum (Smith, L. M. and C. C. Dobson, Applied Optics 28: 3339-3342), wherein the distance to a scatterer is determined by the wavelength dependent fringe spacing within the power spectrum. SD-OCT has enabled the determination of distance and scattering intensity of multiple scatters lying along the illumination axis by analyzing a single the exposure of an array of optical detectors so that no scanning in depth is necessary. Typically the light source emits a broad range of optical frequencies simultaneously.

Alternatively, in swept-source OCT, the interference spectrum is recorded by using a source with adjustable optical frequency, with the optical frequency of the source swept through a range of optical frequencies, and recording the interfered light intensity as a function of time during the sweep. An example of swept-source OCT is described in U.S. Pat. No. 5,321,501.

Generally, time domain systems and frequency domain systems can further vary in type based upon the optical layout of the systems: common beam path systems and differential beam path systems. A common beam path system sends all produced light through a single optical fiber to generate a reference signal and a sample signal whereas a differential beam path system splits the produced light such that a portion of the light is directed to the sample and the other portion is directed to a reference surface. Common beam path systems are described in U.S. Pat. 7,999,938; U.S. Pat. 7,995,210; and U.S. Pat. 7,787,127 and differential beam path systems are described in U.S. Pat. 7,783,337; U.S. Pat. 6,134,003; and U.S. Pat. 6,421,164.

Scope of the invention is thus indicated by the appended claims rather than by the foregoing description, and all changes which come within the meaning and range of equivalency of the claims are therefore intended to be embraced therein.

## Claims

1. A rotational imaging apparatus (300), the apparatus comprising:
a hollow elongate transducer shaft (304), wherein a proximal end of the transducer shaft comprises a connector (228); an elongate catheter (302), wherein the transducer shaft is configured to fit within the catheter;
a rotatable elongate drive member (322) within the transducer shaft;
a rotatable elongate electrical signal transmission member (323) within the drive member (322); and
an imaging device (318) within the transducer shaft, wherein the drive member (322) and the signal transmission member (323) are coupled to the imaging device (318);
haracterized in that only one portion (324) of the signal transmission member (323) is attached to the drive member (322) distal to the connector, the one portion (324) being at a position immediately after the imaging device (318).

2. The apparatus according to claim 1, comprising a catheter hub (216) near a proximal end portion (206) of the catheter (302) and the proximal end portion (210) of the transducer shaft (304), the catheter hub (216) includes a stationary hub housing (224), a dog (226), the connector (228), and bearings (230), wherein the dog (226) is configured to mate with a spinning element (232) of an interface module for alignment of the hub catheter (216) with the interface module (214) and torque transmission to the transducer shaft (304), wherein the dog (226) is rotatable within the stationary hub housing (224) utilizing the bearings (230), and wherein the connector (228) is rotatable with the spinning element (232).

3. The apparatus according to claim 1, wherein the imaging device (318) comprises an ultrasound transducer.

4. The apparatus according to claim 1, wherein the transducer shaft (304), the drive member (322), and the electrical signal transmission member (323) are flexible.

5. The apparatus according to claim 1, wherein the apparatus is connected to an interface module (114) via the connector, the interface module comprising components configured to rotate the drive member (322) and the electrical signal transmission member (323).

6. The apparatus according to claim 1, wherein the transducer comprises a piezoelectric material.

7. The apparatus according to claim 1, wherein the imaging device (318) is coupled to a distal end of the drive member (322).

8. The apparatus according to claim 1, wherein electrical signal transmission member (323) is attached to the drive member (322) by an adhesive.

9. The apparatus according to claim 1, wherein the electrical signal transmission member (323) is coaxial cable.

10. The apparatus according to claim 1, wherein the transducer shaft (304) is flushable with a sterile fluid within the catheter (302), wherein a fluid injection port is supplied at a junction of the catheter (302) and an interface module so that a space inside the catheter (302) can be flushed.

## Patentansprüche

1. Drehbildgebungsvorrichtung (300), wobei die Vorrichtung umfasst:
eine hohle gestreckte Transducerwelle (304), wobei ein proximales Ende der Transducerwelle ein Verbindungselement (228) umfasst;
einen gestreckten Katheter (302), wobei die Transducerwelle konfiguriert ist, um in den Katheter zu passen;
ein drehbares gestrecktes Antriebsteil (322) innerhalb der Transducerwelle;
ein drehbares gestrecktes elektrisches Signalübertragungsteil (323) innerhalb des Antriebsteils (322); und
eine Bildgebungsvorrichtung (318) innerhalb der Transducerwelle, wobei das Antriebsteil (322) und das Signalübertragungsteil (323) an die Bildgebungsvorrichtung (318) gekoppelt sind;
**dadurch gekennzeichnet, dass**
nur ein Abschnitt (324) des Signalübertragungsteils (323) an das distal zum Verbindungselement liegende Antriebsteil (322) befestigt ist, wobei ein Abschnitt (324) an einer Stelle unmittelbar nach der Bildgebungsvorrichtung (318) liegt.

2. Vorrichtung nach Anspruch 1, umfassend eine Katheternabe (216) in der Nähe eines proximalen Endabschnitts (206) des Katheters (302) und des proximalen Endabschnitts (210) der Transducerwelle (304), die Katheternabe (216) enthält ein ortsfestes Nabengehäuse (224), einen Auflagebock (226), das Verbindungselement (228) und Lager (230), wobei der Auflagebock (226) konfiguriert ist, um sich an ein kreiselndes Element (232) eines Schnittflächenmoduls für die Ausrichtung des Nabenkatheters (216) mit dem Schnittflächenmodul (214) und Drehmomentübertragung an die Transducerwelle (304) anzukuppeln, wobei der Auflagebock (226) unter Verwendung der Lager (230) drehbar innerhalb des ortsfesten Nabengehäuses (224) ist, und wobei das Verbindungselement (228) drehbar innerhalb des kreiselnden Elements (232) ist.

3. Vorrichtung nach Anspruch 1, wobei die Bildgebungsvorrichtung (318) einen Ultraschall-Transducer umfasst.

4. Vorrichtung nach Anspruch 1, wobei die Transducerwelle (304) das Antriebsteil (322) und das elektrische Signalübertragungsteil (323) beweglich sind.

5. Vorrichtung nach Anspruch 1, wobei die Vorrichtung über das Verbindungselement mit einem Schnittflächenmodul (114) verbunden ist, wobei das Schnittflächenmodul Komponenten umfasst, die konfiguriert sind, um das Antriebsteil (322) und das elektrische Signalübertragungsteil (323) zu drehen.

6. Vorrichtung nach Anspruch 1, wobei der Transducer ein piezoelektrisches Material umfasst.

7. Vorrichtung nach Anspruch 1, wobei die Bildgebungsvorrichtung (318) an ein distales Ende des Antriebsteils (322) gekoppelt ist.

8. Vorrichtung nach Anspruch 1, wobei das elektrische Signalübertragungsteil (323) durch ein Klebemittel an das Antriebsteil (322) befestigt ist.

9. Vorrichtung nach Anspruch 1, wobei das elektrische Signalübertragungsteil (323) Koaxialkabel ist.

10. Vorrichtung nach Anspruch 1, wobei die Transducerwelle (304) mit einer sterilen Flüssigkeit innerhalb des Katheters (302) ausspülbar ist, wobei eine Flüssigkeitseinspritzöffhung an einer Verbindungsstelle des Katheters (302) bereitgestellt wird und wobei ein Schnittflächenmodul so ist, dass ein Raum innerhalb des Katheters (302) ausgespült werden kann.

## Revendications

1. Appareil d'imagerie rotatif (300), l'appareil comprenant :
un arbre de transducteur (304) allongé creux, dans lequel une extrémité proximale de l'arbre de transducteur comprend un connecteur (228) ;
un cathéter (302) allongé, dans lequel l'arbre de transducteur est configuré pour s'ajuster à l'intérieur du cathéter ;
un élément d'entraînement (322) allongé rotatif à l'intérieur de l'arbre de transducteur ;
un élément de transmission de signal électrique (323) allongé rotatif à l'intérieur de l'élément d'entraînement (322) ; et
un dispositif d'imagerie (318) à l'intérieur de l'arbre de transducteur, dans lequel l'élément d'entraînement (322) et l'élément de transmission de signal (323) sont couplés au dispositif d'imagerie (318) ;
**caractérisé en ce que** seulement une portion particulière (324) de l'élément de transmission de signal (323) est attachée à l'élément d'entraînement (322) de façon distale par rapport au connecteur, la portion particulière (324) étant à une position immédiatement après le dispositif d'imagerie (318).

2. Appareil selon la revendication 1, comprenant une embase de cathéter (216) près d'une portion d'extrémité proximale (206) du cathéter (302) et la portion d'extrémité proximale (210) de l'arbre de transducteur (304), l'embase de cathéter (216) inclut un logement d'embase immobile (224), un chien (226), le connecteur (228) et des paliers (230), dans lequel le chien (226) est configuré pour s'accoupler avec un élément de rotation rapide (232) d'un module d'interface pour l'alignement du cathéter d'embase (216) avec le module d'interface (214) et la transmission de couple à l'arbre de transducteur (304), dans lequel le chien (226) est rotatif à l'intérieur du logement d'embase immobile (224) en utilisant les paliers (230), et dans lequel le connecteur (228) est rotatif avec l'élément de rotation rapide (232).

3. Appareil selon la revendication 1, dans lequel le dispositif d'imagerie (318) comprend un transducteur à ultrasons.

4. Appareil selon la revendication 1, dans lequel l'arbre de transducteur (304), l'élément d'entraînement (322) et l'élément de transmission de signal électrique (323) sont souples.

5. Appareil selon la revendication 1, dans lequel l'appareil est relié à un module d'interface (114) par l'intermédiaire du connecteur, le module d'interface comprenant des composants configurés pour faire tourner l'élément d'entraînement (322) et l'élément de transmission de signal électrique (323).

6. Appareil selon la revendication 1, dans lequel le transducteur comprend une matière piézoélectrique.

7. Appareil selon la revendication 1, dans lequel le dispositif d'imagerie (318) est couplé à une extrémité distale de l'élément d'entraînement (322).

8. Appareil selon la revendication 1, dans lequel l'élément de transmission de signal électrique (323) est attaché à l'élément d'entraînement (322) par un adhésif.

9. Appareil selon la revendication 1, dans lequel l'élément de transmission de signal électrique (323) est un câble coaxial.

10. Appareil selon la revendication 1, dans lequel l'arbre de transducteur (304) est lavable avec un fluide stérile à l'intérieur du cathéter (302), dans lequel un orifice d'injection de fluide est fourni au niveau d'une jonction du cathéter (302) et d'un module d'interface de sorte qu'un espace à l'intérieur du cathéter (302) peut être lavé.
